# EUROPÄISCHE PATENTSCHRIFT

(11) **EP 2 473 477 B1**
(45) Veröffentlichungstag und Bekanntmachung des Hinweises auf die Patenterteilung: **14.01.2015**
(21) Anmeldenummer: 10743186.8
(22) Anmeldetag: 20.08.2010
(51) Int. Cl.: C07C 201/08

(54) **VERFAHREN ZUR HERSTELLUNG VON MONONITRIERTEN ORGANISCHEN VERBINDUNGEN**
PROCESS FOR PREPARING MONONITRATED ORGANIC COMPOUNDS
PROCÉDÉ DE PRODUCTION DE COMPOSÉS ORGANIQUES MONONITRÉS

(30) Priorität: 31.08.2009 EP 09169016
(43) Veröffentlichungstag der Anmeldung: 11.07.2012
(73) Patentinhaber: BASF SE, 67056 Ludwigshafen (DE)
(72) Erfinder: DENISSEN, Leo, Brasschaat (BE); STROEFER, Eckhard, 68163 Mannheim (DE); ARNDT, Jan-Dirk, 68167 Mannheim (DE); MATTKE, Torsten, 67251 Freinsheim (DE); HEINEN, Kerstin, 64653 Lorsch (DE); LESCHINSKI, Julia, 68161 Mannheim (DE)
(86) Internationale Anmeldenummer: PCT/EP2010/062176
(87) Internationale Veröffentlichungsnummer: WO 2011/023638

(56) Entgegenhaltungen:
- WO-A2-01/64333

## Beschreibung

Die vorliegende Erfindung betrifft ein Verfahren zur kontinuierlichen Herstellung einer mononitrierten organischen Verbindung, insbesondere ein Verfahren zur Herstellung von Mononitrobenzol. Die Erfindung betrifft insbesondere ein verbessertes kontinuierliches adiabatisches Verfahren zur Herstellung von Nitrobenzol.

Verfahren zur Nitrierung von Aromaten, insbesondere zur Nitrierung von Benzol, sind seit langem bekannt und werden seit vielen Jahren kommerziell durchgeführt, um beispielsweise Mononitrobenzol zu erzeugen, das besonders in der Produktion von Anilin verwendet wird. Üblicherweise umfasst die Herstellung von Nitrobenzol die Zugabe von Salpetersäure und Schwefelsäure zu Benzol. Diese Nitrierung kann im Temperaturbereich von 60 bis 70°C unter Ableitung der Reaktionswärme durchgeführt werden, oder sie kann unter adiabatischen Bedingungen durchgeführt werden. Zur Durchführung der Reaktion werden dabei Salpetersäure und Schwefelsäure vermischt und Benzol zudosiert, das mit der Salpetersäure zu Wasser und im Wesentlichen Nitrobenzol reagiert.

Die Reaktionszone, in der Benzol und Salpetersäure umgesetzt werden, kann aus einer Anordnung von Rührkesseln oder einem Rohrreaktor bestehen, wobei eine gute Durchmischung für die Reaktion erforderlich ist. Bevorzugt wird daher ein Rohrreaktor eingesetzt, in dem mehrere Dispergierelemente über die Länge des Rohrreaktors verteilt angeordnet sind, die eine intensive Durchmischung von Benzol, Salpetersäure, Schwefelsäure und Wasser gewährleisten. Ein derartiger Reaktor ist beispielsweise in der WO 2001/64333 A2 beschrieben.

Das im Wesentlichen salpetersäurefreie Reaktionsgemisch, das nach der Reaktion erhalten wird, wird einem Phasentrennapparat zugeführt, in dem sich zwei Phasen bilden, wobei die erste als Rohnitrobenzol bezeichnet wird und im Wesentlichen aus Nitrobenzol, Benzol und einer im Nitrobenzol gelösten Menge an Schwefelsäure und Wasser besteht, und die zweite als Absäure bezeichnete im Wesentlichen aus Wasser, Schwefelsäure und in der Schwefelsäure gelöstem Nitrobenzol besteht.

Da während der Umsetzung die Säurephase und die Benzolphase nicht mischbar sind, sind die Reaktionsgeschwindigkeiten und die Raum-Zeit-Ausbeute in starkem Maße durch den Stoffübergang zwischen den Phasen begrenzt, d.h., durch die Fähigkeit, große Grenzflächenbereiche jeder der Phasen zueinander zu exponieren. Wenn die Grenzflächenbereiche vergrößert werden, wird die Reaktionsgeschwindigkeit zwischen den Phasen erhöht. In üblichen Nitrobenzol-Produktionsanlagen werden diese Grenzflächenbereiche durch Umsetzen der Reaktionsteilnehmer in einem oder mehreren durchmischten Gefäßen geschaffen, wo hohe Scherkräfte auf die Flüssigkeiten aufgebracht werden.

Bei der industriellen Nitrierung von Benzol fallen neben dem Hauptprodukt, dem Nitrobenzol, auch zahlreiche Nebenprodukte in der Größenordnung von einigen 100 ppm bis einigen 1000 ppm an. Die auftretenden Nebenprodukte lassen sich in zwei Gruppen unterteilen: mehrfach nitrierte Benzole und phenolische Nebenprodukte, die einfach bzw. mehrfach nitriert sind. Als mehrfach nitrierte Benzole treten die isomeren Dinitrobenzole 1,2-Dinitrobenzol, 1,3-Dinitrobenzol und 1,4-Dinitrobenzol auf. Die gebildeten phenolischen Nebenprodukte sind die isomeren nitrierten Phenole 2-Nitrophenol, 3-Nitrophenol und 4-Nitrophenol. Ebenso fallen auch die isomeren dinitrierten Phenole 2,4-Dinitrophenol und 2,6-Dinitrophenol sowie das dreifach nitrierte 2,4,6-Trinitrophenol (Pikrinsäure) an. Obwohl die Nebenprodukte nur in geringen Konzentrationen anfallen, führen sie aufgrund ihrer hohen Toxizität und Ökotoxizität sowie ihrer aufwändige Abtrennbarkeit zu einem erhöhten Aufwand bei der Produkt- und Abwasseraufbereitung und beeinträchtigen daher die Wirtschaftlichkeit des Nitrobenzolverfahrens sehr stark.

Zur Vermindung des Auftretens der vorstehend genannten Nebenprodukte wird üblicherweise ein Überschuss von Benzol eingesetzt und ein besonders ausgestalteter Reaktor eingesetzt, um insbesondere die Bildung von mehrfach nitrierten Benzolen zu unterdrücken. Hierzu wird beispielsweise in der WO 01/46433 A2 vorgeschlagen, einen Rohrreaktor zu verwenden, in dem sich Dispergier- mit Ruhezonen abwechseln. Ein Nachteil dieses Reaktors ist jedoch eine starke Separation der Phasen (Sedimentation) in den Ruhezonen des Reaktors, was einer sehr energieaufwendigen Vermischung der einzelnen Reaktanden in den daran anschließenden Dispergierzonen bedarf.

Aufgabe der vorliegenden Erfindung ist es, ein Verfahren zur kontinuierlichen Herstellung von nitrierten Aromaten, insbesondere zur Herstellung von Nitrobenzol, zur Verfügung zu stellen, das in einem einfach aufgebauten Rohrreaktor durchgeführt werden kann, und einen hohen Endumsatz sowie eine geringe Menge an Nebenprodukten liefert.

Gelöst wird diese Aufgabe durch ein Verfahren zur kontinuierlichen Herstellung von nitrierten Aromaten durch Umsetzung mindestens eines Aromaten mit einem Salpetersäure, Schwefelsäure und Wasser enthaltenden Gemisch, umfassend die Schritte
(a) Einspeisen des mindestens einen Aromaten und des Salpetersäure, Schwefelsäure und Wasser enthaltenden Gemisches in beliebiger Reihenfolge in einen Rohrreaktor, wobei der Rohrreaktor mehrere vertikal angeordnete Rohrbögen mit einem Umlenkwinkel im Bereich von 160° bis 200°, die über die Gesamtlänge des Reaktors in einem Abstand, der dem 30- bis 70-fachen Durchmesser des Reaktors entspricht, angeordnet sind, sowie eine Anzahl statischer Mischelemente, die derart angeordnet sind,
   dass einem ersten Teil des Rohrreaktors Bedingungen vorherrschen, unter denen die Bodensteinzahl einen Wert, bzw. Werte kleiner als 5 annimmt
   und in einem zweiten Teil des Reaktors Bedingungen vorherrschen, unter denen die Bodensteinzahl einen Wert, bzw. Werte größer als 5 annimmt
(b) Umsetzung des mindestens einen Aromaten und des Salpetersäure, Schwefelsäure und Wasser enthaltenden Gemisches in dem Reaktor unter adiabatischen Bedingungen, um ein Reaktionsgemisch zu erhalten, und
(c) Trennung des in Schritt (b) erhaltenen Reaktionsgemisches nach Durchführung der Reaktion in eine organische und eine wässrige Phase.

Überraschend stellte sich heraus, dass mit Hilfe des erfindungsgemäßen Verfahrens aromatische Mononitroverbindungen, insbesondere Nitrobenzol, in hoher Ausbeute, mit geringem Anteil an Nebenprodukten hergestellt werden können, ohne dass aufwendige und komplizierte Reaktoren eingesetzt werden müssen.

Durch Einsatz des Rohrreaktors, der mehrere vertikal angeordnete Rohrbögen mit einem Umlenkwinkel im Bereich von 160° bis 200°, bevorzugt mit einem Umlenkwinkel von 180°, die über die Gesamtlänge des Reaktors in einem Abstand, der dem 30- bis 70-fachen, bevorzugt dem 60- bis 40-fachen, besonders bevorzugt dem 45- bis 55-fachen Durchmesser des Reaktors entspricht, angeordnet sind umfasst, wird mit nahezu minimalem apparativen Aufwand, sowie geringer Energiezufuhr eine sehr wirkungsvolle Durchmischung der eingesetzten Edukte, des mindestens einen Aromaten und des Salpetersäure, Schwefelsäure und Wasser enthaltenden Gemisches, erreicht, wodurch gleichzeitig in vorteilhafter Weise eine hohe Dispergierwirkung und somit eine hohe Reaktionsgeschwindigkeit erreicht wird. Unter dem Begriff "vertikal angeordneter Rohrbogen" ist im Rahmen der Erfindung ein Rohrbogen zu verstehen, der vertikal zum Untergrund ausgerichtet ist und der somit der Redispergierung des Zweiphasengemisches dient. Vorteilhafterweise wird das Zweiphasengemisch während es den vertikal angeordneten Rohrbogen durchströmt mit minimalem apparativen Aufwand sehr wirkungsvoll durchmischt.

Die Anordnung der Rohrbögen in dem Rohrreaktor wird in Abhängigkeit der Reaktionsbedingungen vorgenommen. Gemäß einer Ausführungsform der Erfindung wird der Rohrreaktor durch die Bögen in 6 bis 18 Kammern, besonders bevorzugt in 10 bis 12 Kammern, eingeteilt. Gemäß einer weiteren Ausführungsform der Erfindung sind die Rohrbögen über die Gesamtlänge des Rohrreaktors äquidistant angeordnet.

Der Rohrreaktor, in dem erfindungsgemäße Verfahren durchgeführt wird, umfasst ferner eine Anzahl statischer Mischelemente, die derart angeordnet sind, dass im Zusammenspiel mit den Rohrbögen in einem ersten Teil des Rohrreaktors eine hohe Rückvermischung und in einem zweiten Teil des Rohrreaktors eine geringere Rückvermischung vorherrscht. Die Länge des ersten Teils des Rohrreaktors, in dem eine hohe Rückvermischung vorherrscht, beträgt 20 bis 40% der Gesamtlänge des Rohrreaktors, bevorzugt 25 bis 35% bezogen auf die Gesamtlänge des Rohrreaktors, die Länge des zweiten Teils des Rohrreaktors, in dem eine geringere Rückvermischung vorherrscht, beträgt 60 bis 80%, bevorzugt 65 bis 75%, bezogen auf die Gesamtlänge des Rohrreaktors. Die statischen Mischelemente sind dabei so angeordnet, dass in den entsprechenden Abschnitten die vorstehend definierten Bedingungen vorherrschen. "Hohe Rückvermischung" bedeutet im Sinne der Erfindung, dass in diesem Teilbereich des Reaktors Bedingungen vorherrschen, unter denen die Bodensteinzahl einen Wert, bzw. Werte kleiner als 5 annimmt. "Geringere Rückvermischung" bedeutet, dass in diesem Teilbereich des Reaktors Bedingungen vorherrschen, unter denen die Bodensteinzahl einen Wert, bzw. Werte größer als 5 annimmt. Die Anordnung der Rohrbögen und der statischen Mischelemente kann von dem Fachmann auf Basis seines Fachwissens derart vorgenommen werden, dass in den Entsprechenden Bereichen des Reaktors die entsprechenden Bedingungen vorherrschen.

Als statische Mischelemente weist der Rohrreaktor bevorzugt Blenden, insbesondere versetzt angeordnete Blenden, Schüttungen und Böden mit geeigneten Öffnungen auf. Da die bei der adiabatischen Verfahrensführung auftretenden hohen Reaktionstemperaturen und die aggressiven Einsatzstoffe an das Material der Böden hohe Anforderungen stellen, wird für die Böden ein unter diesen Bedingungen nahezu inertes Material verwendet, besonders bevorzugt werden Edelstahl- bzw. Tantalböde sowie beschichtete oder keramische Werkstoffe verwendet.

Die Böden fungieren als Dispergierelemente, und weisen Öffnungen auf. Bei den Öffnungen kann es sich um Schlitze, Löcher oder Bohrungen handeln. Besonders bevorzugt handelt es sich bei den Öffnungen um Bohrungen, da ihre Fertigung besonders einfach zu bewerkstelligen ist. Es können jedoch auch andere Öffnungsformen gewählt werden. Gemäß einer Ausführungsform der Erfindung hat es sich als vorteilhaft herausgestellt, Böden einzusetzen, die als Lochböden ausgestaltet sind. Die Lochböden weisen dabei eine Vielzahl von Löchern auf, die gemäß einer Ausführungsform der Erfindung symmetrisch bzw. gleichmäßig über die Fläche des Bodens verteilt sind.

Gemäß einer weiteren Ausführungsform der Erfindung weisen die Lochböden Bereiche auf, in denen ausgehend von einer gleichmäßigen Verteilung der Löcher pro Flächeneinheit eine geringere Anzahl Löcher pro Flächeneinheit vorgesehen ist.

Gemäß einer besonders bevorzugten Ausführungsform der Erfindung hat es sich als vorteilhaft herausgestellt, im Rahmen des erfindungsgemäßen Verfahrens Böden einzusetzen bzw. zu verwenden, die sowohl Bohrungen als auch drei- und/oder vier- und/oder fünf- und/oder sechseckige bzw. segmentartige Aussparungen aufweisen. Die Aussparungen sind dabei über einen Tei der Fläche des Bodens bevorzugt gleichmäßig verteilt, während die Aussparungen über einen anderen Teil der Fläche des Bodens verteilt angeordnet sind. Die drei- und/oder vier- und/oder fünf- und/oder sechseckige bzw. segmentartigen Aussparungen sind gemäß einer Ausführungsform der Erfindung nur teilweise ausgestanzt und kennzeichnen sich dadurch, dass die Ausstanzungen aus der Bodenebene, bevorzugt in Strömungsrichtung gebogen sind und als Strömungsleitbleche fungieren. Der Einsatz derartiger Böden bewirkt eine besonders vorteilhafte Durchmischung der in dem erfindungsgemäßen Verfahren eingesetzten Edukte.

Der im Rahmen des erfindungsgemäßen Verfahrens eingesetzte Reaktor ist bevorzugt vertikal ausgerichtet und weist ein unteres Ende und ein oberes Ende auf. Der Reaktor weist am unteren Ende mindestens eine Zufuhrmöglichkeit zum Einlass der Reaktanden, des mindestens einen Aromaten und des Salpetersäure, Schwefelsäure und Wasser enthaltenden Gemisches, auf und am oberen Ende mindestens eine Abfuhrmöglichkeit zur Entnahme des Reaktionsgemisches. Die Zufuhr der Reaktanden geschieht gemäß einer Ausführungsform der Erfindung mit Hilfe mindestens einer Pumpe. In einer bevorzugten Ausführungsform weist der Reaktor weiterhin Zufuhrmöglichkeiten für die organische und wässrige Phase auf, welche eine Zufuhr in die einzelnen im Reaktor befindlichen Kammern ermöglicht.

Die Reaktionsteilnehmer können gemeinsam, jedoch auch einzeln oder als Gemische zweier oder dreier von ihnen gleichzeitig oder sukzessive dem Reaktor zugeführt werden. Gemäß einer Ausführungsform der Erfindung können die Reaktionsteilnehmer in der vorstehend genannten Art und Weise über eine oder mehrere Zuführung(en), wie Düse(n) eingebracht werden.

Für das Gelingen der Reaktion ist es dabei von geringer Bedeutung, in welcher Reihenfolge und Zusammensetzung die Reaktionsteilnehmer Salpetersäure, der Aromat sowie Schwefelsäure und Wasser miteinander vermischt werden, solange das resultierende Reaktionsgemisch nach der Gesamtvermischung die erfindungsgemäße Zusammensetzung besitzt.

Um über den gesamten Reaktor eine unerwünschte Rückströmung durch die Einbauten, insbesondere Böden zu vermeiden, werden in dem Rohrreaktor bevorzugt Böden eingesetzt, welche einen Druckverlust von 0,05 bis 3 bar pro Boden erzeugen. Besonders bevorzugt werden zur adiabatischen Mononitrierung von Aromaten Böden eingesetzt, welche einen Druckverlust von 0,05 bis 1 bar, ganz besonders bevorzugt von 0,08 bis 0,8 bar erzeugen. Vorzugsweise wird der Druckverlust pro Boden möglichst gering gehalten, da zur Bereitstellung eines höheren Druckverlusts beispielsweise eine Pumpe höherer Leistung benötigt wird, die wiederum zu höheren Kosten im Gesamtverfahren führt.

Das Einspeisen der Edukte geschieht gemäß einer Ausführungsform der Erfindung über mindestens eine Zuführung, die es gestattet, die Edukte/Reaktionspartner unter hoher Durchmischung in den Rohrreaktor einzuspeisen. Gemäß einer Ausführungsform der Erfindung kann dabei der mindestens eine Aromat über eine Zuführung, beispielsweise eine Düse und das Salpetersäure, Schwefelsäure und Wasser enthaltende Gemisch über eine weitere Zuführung, beispielsweise eine Düse in den Rohrreaktor eingespeist werden, gemäß einer bevorzugten Ausführungsform können der mindestens eine Aromat sowie das Salpetersäure, Schwefelsäure und Wasser enthaltende Gemisch gleichzeitig über eine Zuführung, beispielsweise über eine Düse in den Rohrreaktor eingespeist werden. Gemäß einer weiteren bevorzugten Ausführungsform wird das Salpetersäure, Schwefelsäure und Wasser enthaltende Gemisch über mehrere Zuführungen, beispielsweise über mehrere Düsen, die über die Länge des Rohrreaktors verteilt angeordnet sind, dem Rohrreaktor zugeführt.

Die Düse(n), die gemäß einer bevorzugten Ausführungsform der Erfindung im Rahmen des erfindungsgemäßen Verfahrens eingesetzt wird bzw. werden, sind dem Fachmann auf dem Gebiet der Reaktortechnik bekannt. Bevorzugt ist die Düse derart ausgestaltet, dass sie fähig ist, einen turbulenten Strom als divergente Übergangsschicht abzugeben, die ein divergentes Muster (Vollkonus) von Tröpfchen produziert und den genannten Tröpfchen ein Drehbewegungselement vermitteln kann oder nicht. Solche Strömungsmuster können durch Verwendung von Düsen, die aus der Technik des Sprühtrocknens bekannt sind, erhalten werden. Die Düse weist bevorzugt innere Wände oder andere Mittel auf, die einen oder mehrere tangentiale oder schraubenförmige Durchgänge definieren, um einen radialen (schraubenförmigen) Austrittsstrom zu schaffen, der einem linearen divergenten Strom überlagert ist, um einen resultierenden schraubenförmigen Strom zu erzeugen, der zur Verstärkung der Dispersion der beim Austritt gebildeten Tröpfchen dient. Die Düse weist bevorzugt einen Austrittskonuswinkel von 70° oder weniger auf.

Die Zuführungen, die im Rahmen des erfindungsgemäßen Verfahrens eingesetzt werden, sind dem Fachmann auf dem Gebiet der Reaktortechnik bekannt. Bevorzugt ist die Zuführung derart ausgestaltet, dass sie bereits eine Vormischung erzeugt. Dies kann durch den Einsatz verschiedener, dem Fachmann bekannten Mischdüsen aber auch statischen Mischern oder einem T-Stück erreicht werden.

Die lineare Fluidgeschwindigkeit durch die Zuführung, insbesondere durch die Düse typischerweise von 5 bis 50 m/s und es werden durchschnittliche Tröpfchengrößen von 10 bis 1000 µm erzielt.

Die Vermischung der Reaktionsteilnehmer in der Zuführung bzw. in dem Reaktor erfolgt im Bereich von 20 bis 110°C, bevorzugt im Bereich von 40 bis 100°C, besonders bevorzugt im Bereich von 55 bis 95°C. Es werden adiabatische Reaktionsbedingungen eingehalten. Die Endtemperatur ist abhängig von der Höhe der Mischtemperatur, von den Mengenverhältnissen der Reaktionsteilnehmer und vom Umsatz; sie übersteigt im Allgemeinen 140°C nicht, meistens nicht 130°C.

Die aromatischen Verbindungen, die bei der vorliegenden Erfindung eingesetzt werden können, sind beispielsweise Benzol, Toluol, Chlorbenzol, Naphthalin und Anthrachinon. Gemäß einer bevorzugten Ausführungsform handelt es sich bei der aromatischen Verbindung um Benzol.

Der Anteil an Salpetersäure in dem Salpetersäure, Schwefelsäure und Wasser enthaltenden Gemisch beträgt bezogen auf die Summe von Salpetersäure, Schwefelsäure und Wasser 1 bis 8 Gew.-%, bevorzugt 2 bis 6 Gew.-%, besonders bevorzugt 2,5 bis 4%. Salpetersäure kann in hoch konzentrierter oder in azeotrop siedender Form, bevorzugt aber in Form der ca. 60 bis 65 Gew.-% aufweisenden und kostengünstig verfügbaren "Schwachsäure" eingesetzt werden.

Der Gehalt an Schwefelsäure in dem Salpetersäure, Schwefelsäure und Wasser enthaltenden Gemisch beträgt, bezogen auf die Summe von Salpetersäure, Schwefelsäure und Wasser 58 bis 74 Gew.-%, bevorzugt 60 bis 70 Gew.-%, besonders bevorzugt 62 bis 68 Gew.-%, ganz besonders bevorzugt 64 bis 67 Gew.-%.

Der Rest zu 100 Gew.-% ist Wasser. Dies kann als solches als Verdünnungs-Wasser der Schwefelsäure, als Verdünnungs-Wasser der Salpetersäure oder in mehreren der genannten Formen eingesetzt werden. In besonders bevorzugter Form liegt Wasser als Verdünnungs-Wasser sowohl der Schwefelsäure als auch der Salpetersäure vor. Das molare Verhältnis des mindestens einen Aromaten, insbesondere des Benzols zu HNO₃ beträgt allgemein 0,9 bis 1,5. Um die Bildung unerwünschter mehrfach nitrierter Aromaten, insbesondere mehrfach nitrierten Benzols zu minimieren, beträgt das Molverhältnis des Aromaten zu Salpetersäure bevorzugt 1,0 bis 1,5, besonders bevorzugt 1,03 bis 1,3, ganz besonders bevorzugt 1,05 bis 1,2. Die Reaktion des erfindungsgemäßen Verfahrens im Falle der Herstellung von Nitrobenzol lautet formelmäßig: C₆H₆ + HNO₃ → O₂N-C₆H₅ + H₂O.

Somit werden gemäß einer bevorzugten Ausführungsform der Erfindung Benzol und HNO₃ in das Verfahren eingeführt und Mononitrobenzol und Wasser ausgeschleust, während das beschriebene H₂SO₄ / H₂O-Gemisch das Reaktionsmedium darstellt. Da bei der technischen Realisierung vorteilhafterweise verdünnte Salpetersäuren eingesetzt werden, muss zusätzlich zum Reaktionswasser auch noch Verdünnungs-Wasser der Salpetersäure ausgeschleust werden.

Als nächstes wird die Fließgeschwindigkeit der Reaktionsteilnehmer beschrieben. Da eine optimale Fließgeschwindigkeit der Reaktionsteilnehmer von verschiedenen Faktoren, wie der Reaktorgröße und den Reaktionsbedingungen abhängt, kann man an dieser Stelle nicht verallgemeinern, jedoch beträgt im Fall eines Rohrreaktors mit einem Innendurchmesser von 50 bis 350 mm die lineare Geschwindigkeit in dem Rohr gewöhnlich 0,20 bis 3,00 m/s, vorzugsweise 0,5 bis 1,5 m/s. Die Verweilzeit der Reaktionsteilnehmer in dem Rohrreaktor hängt von den Reaktionsbedingungen und dem Rohrreaktor ab, beträgt jedoch gewöhnlich 0,5 Minuten bis fünf Minuten, bevorzugt 0,5 Minuten bis 2 Minuten. Der Druck in dem Reaktor beträgt allgemein 2 bis 5 bar abs. Hierdurch wird sichergestellt, dass Benzol unter den vorherrschenden Temperaturbedingungen in flüssiger Form vorliegt.

Länge und Innendurchmesser des Rohrreaktors hängen von den Verfahrensparametern ab, allgemein beträgt die Länge des Rohrreaktors 2,5 bis 300 m, bevorzugt 25 bis 200 m, besonders bevorzugt 100 bis 150 m. Der Innendurchmesser des Reaktors kann ebenfalls über weite Bereiche variieren, beträgt allgemein 50 bis 350 mm, bevorzugt 100 bis 300 mm, besonders bevorzugt 150 bis 280 mm.

Das aus dem Rohrreaktor austretende Reaktionsgemisch wird in Schritt (c) in eine organische Phase und in eine Säurephase in einem Separator aufgetrennt. Die abgetrennte Säurephase wird mit gut bekannten Mitteln, beispielsweise Vorrichtung, zum Beispiel einem Vakuumverdampfer, rekonzentriert, wobei man die durch die Reaktion und das Mischen erzeugte Wärme verwertet und erforderlichenfalls wieder verwendet. Aus der organischen Phase kann die gewünschte aromatische Nitroverbindung gereinigt werden, indem man enthaltene Verunreinigungen durch Anwendung von Verfahren entfernt, die gewöhnlicherweise bei der Nitrierung einer aromatischen Verbindung durchgeführt werden, wie Waschen und Destillation.

Das nachfolgende Beispiel erläutert das erfindungsgemäße Verfahren.

### Beispiel 1

In einem Rohrreaktor mit einem Innendurchmesser d = 200 mm und einer Länge von L = 130 m wird Benzol mit einer Mischsäure bestehend aus 65% H₂SO₄, 3% HNO₃ und 36% Wasser bei einer Anfangstemperatur von T = 90°C unter adiabaten Bedingungen umgesetzt, wobei der molare Überschuss an Benzol 10% beträgt. Der Reaktor ist mit insgesamt 15 statischen Mischelementen ausgestattet, von denen 7 im ersten Drittel, 5 im zweiten Drittel und 3 im dritten Drittel des Reaktorvolumens eingebaut sind. Bei einer Verweilzeit von t = 1,5 min wird eine Nitroaromatenausbeute von 97% erreicht. Der Anteil an phenolischen Nebenkomponenten liegt zwischen 1000 und 5000 ppm. Ist der Reaktor zusätzlich noch mit 12 gleichmäßig über die Länge verteilten Rohrbögen mit einem Radius von 180° ausgestattet, erhöht sich die Ausbeute auf 98%, wobei der Anteil an phenolischen Nebenprodukten um 10 % reduziert werden kann.

## Patentansprüche

1. Verfahren zur kontinuierlichen Herstellung von nitrierten Aromaten durch Umsetzung mindestens eines Aromaten mit einem Salpetersäure, Schwefelsäure und Wasser enthaltenden Gemisch, umfassend die Schritte
(a) Einspeisen des mindestens einen Aromaten und des Salpetersäure-, Schwefelsäure- und Wasser- enthaltende Gemisches in beliebiger Reihenfolge in einen Rohrreaktor, wobei der Rohrreaktor mehrere vertikal angeordnete Rohrbögen mit einem Umlenkwinkel im Bereich von 160° bis 200°, die über die Gesamtlänge des Reaktors in einem Abstand, der dem 30 bis 70-fachen Durchmesser des Reaktors entspricht angeordnet sind, sowie eine Anzahl statischer Mischelemente, die derart angeordnet sind,
dass in einem ersten Teil des Rohrreaktors Bedingungen vorherrschen, unter denen die Bodensteinzahl einen Wert, bzw. Werte kleiner als 5 annimmt
und in einem zweiten Teil des Reaktors Bedingungen vorherrschen, unter denen die Bodensteinzahl einen Wert, bzw. Werte größer als 5 annimmt,
umfasst,
(b) Umsetzung des Reaktionsgemisches in dem Reaktor unter adiabatischen Bedingungen, um ein Reaktionsgemisch zu erhalten, und
(c) Trennung des in Schritt (b) erhaltenen Reaktionsgemisches nach Durchführung der Reaktion in eine organische und eine wässrige Phase.

2. Verfahren nach Anspruch 1, **dadurch gekennzeichnet, dass** die statischen Mischelemente ausgewählt sind aus Schüttungen, Lochblechen, Blenden und statischen Mischern.

3. Verfahren nach Anspruch 1 oder 2, **dadurch gekennzeichnet, dass** man das Salpetersäure, Schwefelsäure und Wasser enthaltende Gemisch über mehrere Zuführungen die über die Länge des Rohrreaktors verteilt angeordnet sind, dem Rohrreaktor zuführt.

4. Verfahren nach einem der Ansprüche 1 bis 3, **dadurch gekennzeichnet, dass** das Molverhältnis von Aromat zu Salpetersäure größer 1,0 bis 1,5, bevorzugt größer 1,03 bis 1,3 beträgt.

5. Verfahren nach einem der Ansprüche 1 bis 4, **dadurch gekennzeichnet, dass** der Rohrreaktor durch die Rohrbögen in 6 bis 18 Kammern unterteilt wird.

6. Verfahren nach einem der Ansprüche 1 bis 5, **dadurch gekennzeichnet, dass** der mindestens eine Aromat aus Benzol, Toluol, Chlorbenzol, Naphthalin und Antrachinon ausgewählt ist.

7. Verfahren nach einem der Ansprüche 1 bis 6, **dadurch gekennzeichnet, dass** der mindestens eine Aromat Benzol ist.

## Claims

1. A process for continuously preparing nitrated aromatics by reacting at least one aromatic with a mixture comprising nitric acid, sulfuric acid and water, comprising the steps of
(a) feeding the at least one aromatic and the mixture comprising nitric acid, sulfuric acid and water in any sequence into a tubular reactor, said tubular reactor having a plurality of vertical tube bends with a deflection angle in the range from 160° to 200°, which are arranged over the total length of the reactor at a distance which corresponds to 30 to 70 times the diameter of the reactor, and a number of static mixing elements which are arranged such that conditions under which the Bodenstein number assumes a value or values greater than 5 exist that in a first part of the tubular reactor and conditions under which the Bodenstein number assumes a value or values greater than 5 exist in a second part of the reactor,
(b) converting the reaction mixture in the reactor under adiabatic conditions in order to obtain a reaction mixture, and
(c) separating the reaction mixture obtained in step (b) after performance of the reaction into an organic phase and an aqueous phase.

2. The process according to claim 1, wherein the static mixing elements are selected from beds, perforated sheets, restrictors and static mixers.

3. The process according to claim 1 or 2, wherein the mixture comprising nitric acid, sulfuric acid and water is fed to the tubular reactor via a plurality of feeds which are arranged distributed over the length of the tubular reactor.

4. The process according to any of claims 1 to 3, wherein the molar ratio of aromatic to nitric acid is greater than 1.0 to 1.5, preferably greater than 1.03 to 1.3.

5. The process according to any of claims 1 to 4, wherein the tubular reactor is divided by the tube bends into 6 to 18 chambers.

6. The process according to any of claims 1 to 5, wherein the at least one aromatic is selected from benzene, toluene, chlorobenzene, naphthalene and anthraquinone.

7. The process according to any of claims 1 to 6, wherein the at least one aromatic is benzene.

## Revendications

1. Procédé de fabrication continue de composés aromatiques nitrés par mise en réaction d'au moins un composé aromatique avec un mélange contenant de l'acide nitrique, de l'acide sulfurique et de l'eau, comprenant les étapes suivantes :
(a) l'introduction du ou des composés aromatiques et du mélange contenant de l'acide nitrique, de l'acide sulfurique et de l'eau dans un ordre quelconque dans un réacteur tubulaire, le réacteur tubulaire comprenant plusieurs coudes verticaux d'un angle de courbure dans la plage allant de 160° à 200°, qui sont agencés sur l'ensemble de la longueur du réacteur à une distance correspondant à 30 à 70 fois le diamètre du réacteur, ainsi qu'un nombre d'éléments de mélange statiques, qui sont agencés de sorte
que des conditions dans lesquelles le nombre de Bodenstein prend une valeur ou des valeurs inférieures à 5 règnent dans une première partie du réacteur tubulaire,
et que des conditions dans lesquelles le nombre de Bodenstein prend une valeur ou des valeurs supérieures à 5 règnent dans une seconde partie du réacteur,
(b) la mise en réaction du mélange réactionnel dans le réacteur dans des conditions adiabatiques pour obtenir un mélange réactionnel et
(c) la séparation du mélange réactionnel obtenu à l'étape (b) après la réalisation de la réaction en une phase organique et une phase aqueuse.

2. Procédé selon la revendication 1, **caractérisé en ce que** les éléments de mélange statiques sont choisis parmi les garnissages, les plaques perforées, les déflecteurs et les mélangeurs statiques.

3. Procédé selon la revendication 1 ou 2, **caractérisé en ce que** le mélange contenant de l'acide nitrique, de l'acide sulfurique et de l'eau est introduit dans le réacteur tubulaire par plusieurs entrées réparties sur la longueur du réacteur tubulaire.

4. Procédé selon l'une quelconque des revendications 1 à 3, **caractérisé en ce que** le rapport molaire entre le composé aromatique et l'acide nitrique est de plus de 1,0 à 1,5, de préférence de plus de 1,03 à 1,3.

5. Procédé selon l'une quelconque des revendications 1 à 4, **caractérisé en ce que** le réacteur tubulaire est divisé par les coudes en 6 à 18 chambres.

6. Procédé selon l'une quelconque des revendications 1 à 5, **caractérisé en ce que** le ou les composés aromatiques sont choisis parmi le benzène, le toluène, le chlorobenzène, la naphtaline et l'anthraquinone.

7. Procédé selon l'une quelconque des revendications 1 à 6, **caractérisé en ce que** le ou les composés aromatiques sont le benzène.
